**Europäisches Patentamt**

**European Patent Office**

**Office européen des brevets**

(11) Veröffentlichungsnummer : **0 023 004**
**B1**

(12) **EUROPÄISCHE PATENTSCHRIFT**

(45) Veröffentlichungstag der Patentschrift :
**13.07.83**

(51) Int. Cl.³ : **F 16 M 11/18, G 02 B 7/00**

(21) Anmeldenummer : **80104050.2**

(22) Anmeldetag : **14.07.80**

(54) **Zusatzvorrichtung an einem Stativ für ein optisches Beobachtungsgerät.**

(30) Priorität : **24.07.79 CH 6849/79**

(43) Veröffentlichungstag der Anmeldung :
**28.01.81 Patentblatt 81/04**

(45) Bekanntmachung des Hinweises auf die Patenterteilung : **13.07.83 Patentblatt 83/28**

(84) Benannte Vertragsstaaten :
**AT BE CH DE FR GB IT LI NL SE**

(56) Entgegenhaltungen :
**CH A 548 568**
**US A 2 967 458**
**US A 3 762 797**

(73) Patentinhaber : **CONTRAVES AG**
**Schaffhauserstrasse 580**
**CH-8052 Zürich (CH)**

(72) Erfinder : **Heller, Rudolf**
**Köschenrütistrasse 12**
**CH-8052 Zürich (CH)**
Erfinder : **Schindler, Walter**
**Carl-Spitteler-Strasse 53**
**CH-8053 Zürich (CH)**

(74) Vertreter : **Althoff, Gerhard et al**
**Patentanwälte H.Mitscherlich, K.Gunschmann**
**Dr.W.Körber, J.Schmidt-Evers Steinsdorfstrasse 10**
**D-8000 München 22 (DE)**

## Zusatzvorrichtung an einem Stativ für ein optisches Beobachtungsgerät

Die Erfindung betrifft eine Zusatzvorrichtung an einem Stativ für ein optisches Beobachtungsgerät, insbesondere für ein Operationsmikroskop, das an dem mit einem Schwenkarm, einem Tragarm, mehreren Dreh- und Bremslagern sowie einem um eine erste Achse drehbaren und um eine quer dazu angeordnete zweite Achse des Tragarms schwenkbaren Gelenkparallelogramm versehenen Stativ angeordnet und in vorbestimmtem räumlichen Bereich hinsichtlich seiner Lage und Orientierung frei stehenbleibend verstellbar ist.

Bei einer als Stativ ausgebildeten Tragvorrichtung für ein Operationsmikroskop der vorstehend geschilderten Art gemäß der CH-A 548 568 ist es bekannt, zum Austausch unterschiedlicher Mikroskope, oder zum Ansetzen verschiedener Hilfsgeräte an das Mikroskop, z. B. Ansetzen einer Film- oder TV-Kamera, auf einem am Schwenkarm angelenkten und im wesentlichen horizontal orientierten Tragarm der Vorrichtung ein Ausgleichsgewicht verschiebbar anzuordnen, mittels welchem die schwerebedingten und an den jeweiligen Dreh- und Bremslagern wirkenden Drehmomente des angehängten Mikroskopes kompensiert werden. Diese Maßnahme zum Kompensieren der Drehmomente mit dem zusätzlichen, je nach Mikroskopgröße und Gewicht relativ weit ausladenden Ausgleichsgewicht eignet sich insbesondere für Tragvorrichtungen schwerer Bauart ohne besondere Raumbegrenzung. Das Ausgleichsgewicht ist jedoch nicht in der Lage, Drehund Kippmomente zu kompensieren, welche durch unterschiedlich schwere Mikroskope oder andere Beobachtungsgeräte am Gelenkparallelogramm dadurch auftreten, daß der Schwerpunkt der am Gelenkparallelogramm angehängten Teile nicht in den drei Drehachsen für die räumliche Bewegung des Mikroskopes bzw. des Beobachtungsgerätes liegt. Die Bremslager sind insbesondere bei schweren Geräten nicht geeignet, diese Dreh- und Kippmomente aufzunehmen.

Der Erfindung liegt die Aufgabe zugrunde, für ein eingangs genanntes Stativ für ein optisches Beobachtungsgerät eine aus verhältnismässig einfachen, im wesentlichen konstruktiven Mitteln gebildete Zusatzvorrichtung zu schaffen, mit deren Hilfe die Schwerpunktlage des am Gelenkparallelogramm angehängten Beobachtungsgerätes bestimmt und somit das Gleichgewicht in Bezug auf die erste Achse schnell und sicher einstellbar ist.

Diese Aufgabe wird durch eine Zusatzvorrichtung an einem Stativ für ein optisches Beobachtungsgerät der eingangs genannten Art dadurch gelöst,

a) an dem am vorderen Ende des Gelenkparallelogramms angeordneten Dreh- und Bremslager ein um die im wesentlichen vertikal orientierte Achse des Dreh- und Bremslagers drehbar gelagertes Kopfstück sowie ein zur Aufnahme und Befestigung des Operationsmikroskopes ausgebildetes Kupplungsstück angeordnet ist,

b) zum Austarieren des am vorderen Ende des Gelenkparallelogramms angehängten Operationsmikroskopes in Bezug auf die vertikale Achse das Kupplungsstück mit einem Kugelgelenk in dem Gehäuse des Kopfstücks beweglich, feststellbar gelagert ist, und

c) zum Austarieren des Operationsmikroskopes in Bezug auf die horizontierte Achse des Tragarmes das Dreh- und Bremslager mit dem Kopfstück und dem Kupplungsstück mit der am vorderen Ende des Gelenkparallelogramms angeordneten Vorrichtung in horizontaler Richtung und vertikaler Richtung verstellbar ist.

Die mit der erfindungsgemäßen Zusatzvorrichtung erreichten Vorteile bestehen darin, daß an dem Gelenkparallelogramm des Stativs nunmehr unterschiedliche Beobachtungsgeräte mit zusätzlichen Film- oder TV-Kameras angehängt werden können und ohne besonderen Aufwand jeweils das Gleichgewicht manuell wieder herstellbar ist. Außerdem ermöglicht die Zusatzvorrichtung unter Beibehaltung einer optimalen Standsicherheit eine baulich kleine und leichte Ausführungsform der als Stativ ausgebildeten Tragvorrichtung.

Die Erfindung wird nachstehend anhand eines Ausführungsbeispiels in Verbindung mit der Zeichnung näher beschrieben. Es zeigt:

Figur 1 ein in Ansicht dargestelltes Schaubild eines Stativs mit einer an einem Gelenkparallelogramm befestigten Zusatzvorrichtung zur Aufnahme eines optischen Beobachtungsgerätes, und

Figur 2 die in grösserem Massstab und teilweise im Schnitt dargestellte Ansicht gemäss Pfeilrichtung X in Fig. 1 der Zusatzvorrichtung.

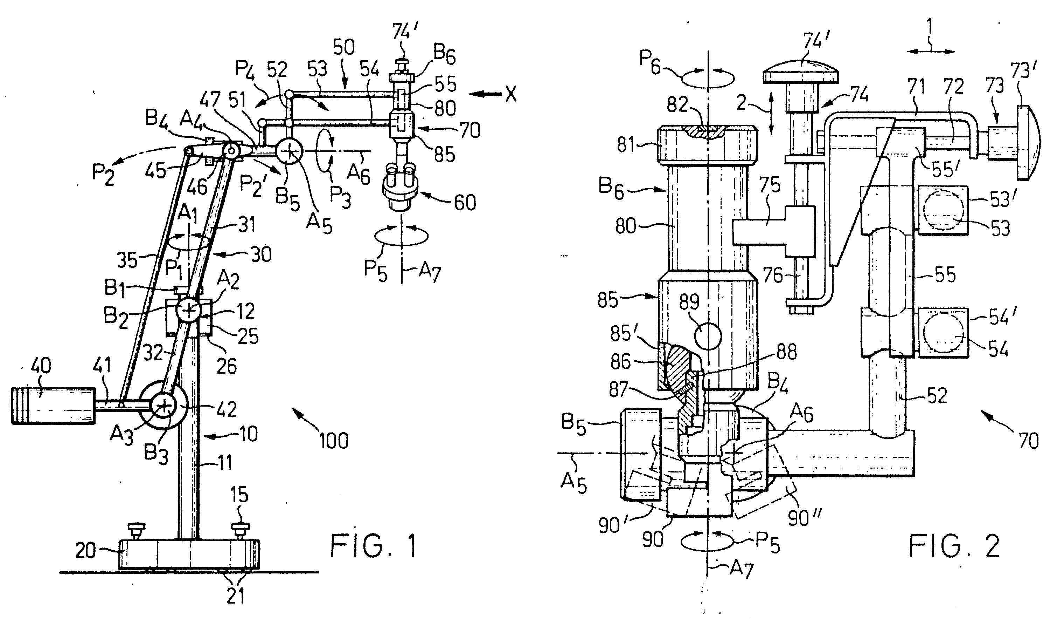

In Fig. 1 ist mit 100 ein mobiles Bodenstativ für Beobachtungsgeräte, insbesondere für Operationsmikroskope in Ansicht dargestellt, welches im wesentlichen eine Standsäule 10, einen aus Teilstücken 31, 32 gebildeten Schwenkarm 30, einen Tragarm 47, sowie ein aus mehreren Gliedern 51, 52, 53 und 54 gebildetes Gelenkparallelogramm 50 aufweist, wobei das Parallelogramm am vorderen Ende zur Aufnahme und Befestigung des schematisch dargestellten Operationsmikroskopes 60 ausgebildet ist.

Die Standsäule 10 besteht im wesentlichen aus einem auf Rollen 21 gelagerten Stativ-Fuss 20, einer vorzugsweise rohrförmigen Säule 11 und einem Kopfstück 12 und ist mittels im Fuss 20 angeordneter Schrauben 15 am Boden nivellier- und arretierbar. Die Rollen 21 sowie die Schrauben 15 sind im Abstand zueinander mit bestimmter geometrischer Aufteilung im Fuss 20 angeordnet. Im oberen Bereich der Säule 11 ist das um eine Vertikalachse $A_1$ gegenüber der feststehenden Säule 11 in Pfeilrichtung $P_1$ drehbare Kopfstück 12 angeordnet, an welchem seitlich ein Bremslager $B_2$ befestigt ist. An dem Bremslager $B_2$ sind die beiden Teilstücke 31, 32 des Schwenkarmes 30 befestigt. Der Schwenkarm 30 ist einerseits um

die Horizontalachse $A_2$ des Bremslagers $B_2$ in Pfeilrichtung $P_2$, $P'_2$ schwenkbar und andererseits zusammen mit dem Kopfstück 12 um die Vertikalachse $A_1$ in Pfeilrichtung $P_1$ drehbar, wobei die Schwenkbewegung $P_2$, $P'_2$ durch das Bremslager $B_2$ und die Drehbewegung $P_1$ durch ein Bremslager $B_1$ manuell abbremsbar sind.

An dem Kopfstück 12 ist am Umfang versetzt zu dem Bremslager $B_2$ ein nicht näher dargestelltes Winkelstück 26 befestigt, welches zur Aufnahme eines Instrumentenkastens 25 für die Stromversorgung des Mikroskopes 60 dient. Der Kasten 25 ist zusammen mit dem Kopfstück 12 und dem Schwenkarm 30 um die Vertikalachse $A_1$ in Pfeilrichtung $P_1$ drehbar.

An dem Teilstück 32 des Schwenkarmes 30 ist im unteren Bereich ein Bremslager $B_3$ und eine Gewichts-Scheibe 42 angeordnet, wobei an dem Bremslager $B_3$ ausserdem eine Stange 41 zur Aufnahme eines Gegengewichts 40 befestigt ist. Das Teilstück 31 des Schwenkarmes 30 weist im oberen Bereich ein nicht näher dargestelltes Lager 46 auf, an welchem ein Schwenkhebel 45 befestigt ist. Der Schwenkhebel 45 sowie die das Gegengewicht 40 tragende Stange 41 sind durch eine im wesentlichen parallel zu dem Schwenkarm 30 verlaufende und an der Stange 41 sowie am Schwenkhebel 45 in nicht näher dargestellter Weise angelenkte Schubstange 35 wirkverbunden. Die Schwenkbewegung $P_2$, $P'_2$ des Armes 30 bewirkt eine Schwenkbewegung des Hebels 45 um die Horizontalachse $A_4$ des Lagers 46 sowie der Stange 41 mit dem Gegengewicht 40 um die Horizontalachse $A_3$ des Bremslagers $B_3$, so dass der Schwenkhebel 45 mit der Stange 41 und die Schubstange 35 mit dem Schwenkarm 30 in jeder Position parallel zueinander verlaufen.

An dem Lager 46 des Schwenkhebels 45 ist seitlich ein Bremslager $B_4$ in nicht näher dargestellter Weise befestigt, in welchem in axialer Richtung der Tragarm 47 in Pfeilrichtung $P_3$ drehbar gelagert ist. Am Ende des Tragarmes 47 ist ein weiteres, quer zum Bremslager $B_4$ angeordnetes Bremslager $B_5$ vorgesehen.

Das aus den gelenkig miteinander verbundenen Gliedern 51, 52, 53 und 54 bestehende Parallelogramm 50 ist mit dem Glied 51 am Tragarm 47 und mit dem Glied 52 am Bremslager $B_5$ in nicht näher dargestellter Weise angelenkt. Die beiden Glieder 53 und 54 des Parallelogramms weisen an ihren Enden je ein Gelenkstück 53', 54' auf, welche durch einen Steg 55 miteinander verbunden sind (Fig. 2). Das Parallelogramm 50 ist mit den am Tragarm 47 angelenkten Gliedern 51, 52 um die Drehachse $A_6$ des Tragarmes 47 in Pfeilrichtung $P_3$ drehbar und um die quer zur Achse $A_6$ orientierte Achse $A_5$ in Pfeilrichtung $P_4$ schwenkbar sowie mittels dem jeweils zugeordneten Bremslager $B_4$ beziehungsweise $B_5$ abbremsbar.

In Fig. 2 ist in Ansicht gemäss Pfeilrichtung X in Fig. 1 eine im vorderen Bereich des Parallelogramms 50 am Steg 55 angeordnete Zusatzvorrichtung 70 dargestellt, welche nachstehend näher beschrieben wird:

Der Steg 55 weist im oberen Bereich ein nicht näher dargestelltes Lager 55' auf, in welchem ein die Zusatzvorrichtung tragender Bolzen 72 gelagert ist. Die Zusatzvorrichtung besteht im wesentlichen aus einem abgewinkelten Tragelement 71, einer mit dem Bolzen 72 wirkverbundenen Stellschraube 73 mit Griff 73', einem seitlich an dem Tragelement 71 angeordneten und gelagerten Bolzen 76, welcher mit einer einen Griff 74' aufweisenden Stellschraube 74 wirkverbunden ist. An dem Bolzen 76 ist eine Halterung 75 angeordnet, an welcher ein Gehäuse 80 eines weiteren Bremslagers $B_6$ befestigt ist. Ferner ist mit gleicher Achsrichtung $A_7$ in dem Gehäuse 80 des Bremslagers $B_6$ ein Kopfstück 85 in nicht näher dargestellter Weise drehbar gelagert.

Das Gehäuse 85' des Kopfstückes 85 ist an dem anderen, teilweise im Schnitt dargestellten Ende für die Lagerung eines Kugelgelenkstückes 86 ausgebildet. In das Gelenkstück 86 ist ein mit einem Gewindestück 88 versehenes und zur Aufnahme und Befestigung des Mikroskopes 60 ausgebildetes Kupplungsstück 90 eingeschraubt und durch eine Stiftschraube 87 fixiert. Das Kupplungsstück 90 ist durch das Kugelgelenk 86 für die Schwerpunktsbestimmung des angehängten Mikroskopes in einem begrenzten, räumlichen Bereich — wie in Fig. 2 durch die verschwenkten Teilstücke 90', 90" des Kupplungsstückes 90 schematisch dargestellt — frei beweglich in dem Gehäuse 85' des Kopfstückes 85 gelagert und durch eine nicht näher dargestellte, am Gehäuse 85' angeordnete Stellschraube 89 in der ermittelten Schwerpunktslage feststellbar.

Das am Kupplungsstück 90 angehängte und in seiner Schwerpunktslage fixierte Mikroskop (in Fig. 2 nicht dargestellt) kann mit dem im Bremslager $B_6$ drehbar gelagerten Kopfstück 85 um die im wesentlichen vertikal orientierte Achse $A_7$ in Pfeilrichtung $P_5$ gedreht werden, wobei die Bremswirkung der Drehbewegung $P_5$ durch einen im Bremslager $B_6$ vorgesehenen und durch die Drehbewegung $P_6$ der Einstellkappe 81 regulierbaren Bremsmechanismus einstellbar ist.

Wie aus Fig. 2 weiterhin ersichtlich, ist im Zentrum der teilweise im Schnitt dargestellten Einstellkappe 81 des Bremslagers $B_6$ ein schematisch dargestelltes, beispielsweise als Dosen-Libelle 82 ausgebildetes Nivellier-Element eingesetzt.

Für den Gewichtsausgleich des am Schwenkarm 47 angelenkten Parallelogramms 50 mit angehängtem Mikroskop 60 wird das Bremslager $B_6$ mit dem Kopfstück 85 und Kupplungsstück 90 mittels der Libelle 82 — die Blase in der Libelle wird konzentrisch im Kreis eingespielt — ausbalanciert und danach wird das Mikroskop 60 mittels dem Kugelgelenk 86 in seine Schwerpunktslage gebracht und fixiert. Anschliessend werden zur Verhinderung eventuell auftretender Kippbewegungen des Gelenkparallelogramms 50 mittels der Stellschraube 73, 73' und dem Bolzen 72 das Bremslager $B_6$ sowie die Teile 85, 90 und 60 miteinander in Bezug auf

die horizontierte Achse A₆ des Tragarmes 47 in Pfeilrichtung 1 horizontal und mittels der Stellschraube 74, 74' und dem Bolzen 76 in Pfeilrichtung 2 vertikal verstellt.

An dieser Stelle sei darauf hingewiesen, dass die an den jeweiligen Gelenkstellen des Stativs 100 angeordneten Bremslager B₁, B₂, B₃, B₄, B₅ und B₆ als Dreh- und Bremslager ausgebildet sind und jedes einzelne Bremslager manuell einstellbar ist, so dass die auf die Dreh- und/oder Schwenkbewegung der einzelnen Trag- und Schwenkarme wirkende Bremskraft beliebig den Erfordernissen einstellbar ist. Der Bremsbelag der nicht dargestellten Bremsmechanismen der voran genannten Bremslager weist vorzugsweise einen Haftreibungskoeffizienten auf, welcher möglichst nahe dem Wert des Gleitreibungskoeffizienten liegt. Als besonders vorteilhaft hat sich ein aus Leder hergestellter Bremsbelag herausgestellt.

**Ansprüche**

1. Zusatzvorrichtung an einem Stativ für ein optisches Beobachtungsgerät, insbesondere für ein Operationsmikroskop (60), das an dem mit einem Schwenkarm (30), einem Tragarm (47), mehreren Dreh- und Bremslagern sowie einem um eine erste Achse (A₆) drehbaren und um eine quer dazu angeordnete zweite Achse (A₅) des Tragarms schwenkbaren Gelenkparallelogramm (50) versehenen Stativ (100) angeordnet und in vorbestimmtem räumlichen Bereich hinsichtlich seiner Lage und Orientierung frei stehenbleibend verstellbar ist, dadurch gekennzeichnet, dass

a) an dem am vorderen Ende des Gelenkparallelogramms (50) angeordneten Dreh- und Bremslager (B₆) ein um die im wesentlichen vertikal orientierte Achse (A₇) des Dreh- und Bremslagers drehbar gelagertes Kopfstück (85) sowie ein zur Aufnahme und Befestigung des Operationsmikroskopes (60) ausgebildetes Kupplungsstück (90) angeordnet ist,

b) zum Austarieren des am vorderen Ende des Gelenkparallelogramms (50) angehängten Operationsmikroskopes (60) in Bezug auf die vertikale Achse (A₇) das Kupplungsstück (90) mit einem Kugelgelenk (86) in dem Gehäuse (85') des Kopfstücks (85) beweglich, feststellbar gelagert ist, und

c) zum Austarieren des Operationsmikroskopes (60) in Bezug auf die horizontierte Achse (A₆) des Tragarmes (47) das Dreh- und Bremslager (B₆) mit dem Kopfstück (85) und dem Kupplungsstück (90) mit der am vorderen Ende des Gelenkparallelogramms (50) angeordneten Vorrichtung (70) in horizontaler Richtung (1) und vertikaler Richtung (2) verstellbar ist.

2. Zusatzvorrichtung nach Anspruch 1, dadurch gekennzeichnet, dass stirnseitig und im Zentrum der Einstellkappe (81) des Dreh- und Bremslagers (B₆) ein als Libelle ausgebildetes Nivellierelement (82) angeordnet ist.

3. Zusatzvorrichtung nach Anspruch 1, dadurch gekennzeichnet, dass am vorderen Ende des Gelenkparallelogramms (50) ein mit den Gliedern (53, 54) wirkverbundener und zur Lagerung der Vorrichtung (70) ausgebildeter Steg (55) angeordnet ist.

4. Zusatzvorrichtung nach Anspruch 1 und 3, dadurch gekennzeichnet, dass die Vorrichtung (70) ein im wesentlichen an dem Steg (55) gelagertes Tragelement (71) sowie eine zur Lagerung des Dreh- und Bremslagers (B₆) ausgebildete Halterung (75) umfasst, und dass das Tragelement (71) in Bezug auf die Achse (A₆) durch eine erste Stellschraube (73, 73') in horizontaler Richtung (1) und durch eine zweite Stellschraube (74, 74') in vertikaler Richtung (2) verstellbar ist.

**Claims**

1. Additional arrangement on a support for an optical observation apparatus, in particular for an operational microscope (60) which is disposed on the support (100) provided with a swivel arm (30), a supporting arm (47), a plurality of pivot- and stop-bearings and a swivellable jointed parallelogram (50) rotatable round a first axis (A₆) and round a second axis (A₅) of the supporting arm arranged at right angles to it, and is freely fixedly adjustable in a predetermined spatial area with respect to its position and orientation, characterised in that

a) on the pivot- and stop-bearing (B₆) arranged at the front end of the jointed parallelogram (50), there is disposed a head piece (85) rotatably mounted round the substantially vertically oriented axis (A₇) of the pivot- and stop-bearing, and a coupling piece (90) constructed for receiving and securing the operational microscope (60),

b) to calibrate the operational microscope (60) connected at the front end of the jointed parallelogram (50) with respect to the vertical axis (A₇), the coupling piece (90) with a ball and socket joint (86) is mounted movably and fixably in the housing (85') of the head piece (85), and

c) to calibrate the operational microscope (60) with respect to the levelled axis (A₆) of the support arm (47), the pivot- and stop-bearing (B₆) with the head piece (85) and the coupling piece (90) with the device (70) disposed at the front end of the jointed parallelogram (50) is adjustable in the horizontal direction (1) and the vertical direction (2).

2. Additional arrangement according to claim 1, characterised in that on the front and in the centre of the adjusting cap (81) of the pivot- and stop-bearing (B₆) there is disposed a levelling element (82) formed as a spirit level.

3. Additional arrangement according to claim 1, characterised in that at the front end of the jointed parallelogram (50) there is disposed a crosspiece (55) operatively connected with the elements (53, 54) and constructed to support the device (70).

4. Additional arrangement according to claim 1 and 3, characterised in that the device (70) incorporates a supporting element (71) mounted substantially on the crosspiece (55) and a holder (75) constructed to support the pivot- and stop-bearing (B$_6$), and in that the supporting element (71) can be adjusted with respect to the axis (A$_6$) by a first adjustment screw (73, 73') in the horizontal direction (1) and by a second adjustment screw (74, 74') in the vertical direction (2).

## Revendications

1. Dispositif additionnel prévu sur le support d'un appareil d'observation optique notamment d'un microscope d'opérations (60) prévu sur un support (100) muni d'un bras pivotant (30), d'un bras de support (47), de plusieurs paliers de rotation et de frein ainsi que d'un parallélogramme articulé (50) tournant autour d'un premier axe (A$_6$) et d'un second axe (A$_5$) placé transversalement au précédent et qui peut se régler dans un volume d'espace, prédéterminé quant à sa position et à son orientation en restant librement en place, dispositif caractérisé en ce que :

a) une pièce de tête (85) est montée à rotation autour de l'axe d'orientation essentiellement vertical (A$_7$) du palier de rotation et de freinage (B$_6$) ainsi qu'une pièce d'accouplement (90) prévue pour recevoir et fixer le microscope d'opérations (60) sont prévues sur le palier de rotation et de freinage (B$_6$) prévu à l'extrémité avant du parallélogramme articulé (50) ;

b) pour équilibrer le microscope d'opérations (60) accroché à l'extrémité avant du parallélogramme articulé (50), par rapport à l'axe vertical (A$_7$), la pièce d'accouplement (90) est montée mobile dans le boîtier (85') de la pièce de tête (85) par l'intermédiaire d'une articulation à rotule (86) susceptible d'être bloquée, et

c) pour équilibrer le microscope d'opérations (60) par rapport à l'axe horizontal (A$_6$) du bras de support (46), le palier de rotation et de frein (B$_6$) avec la pièce de tête (85) et la pièce d'accouplement (90) est réglable en direction horizontale (1) et en direction verticale (2) par le dispositif (70) prévu à l'extrémité avant du parallélogramme articulé (50).

2. Dispositif additionnel selon la revendication 1, caractérisé en ce que frontalement et au centre du capuchon de réglage (81) du palier de rotation et de frein (B$_6$) se trouve un élément de mise à niveau (82) en forme de niveau à bulle.

3. Dispositif additionnel selon la revendication 1, caractérisé en ce qu'à l'extrémité avant du parallélogramme articulé (50), est prévue une entretoise (50) reliée de façon à coopérer avec les organes (53, 54) et une entretoise (55) pour servir de palier au dispositif (70).

4. Dispositif additionnel selon les revendications 1 et 3, caractérisé en ce que le dispositif (70) comporte un élément de support (71) essentiellement monté sur l'entretoise (55) ainsi qu'un moyen de fixation (75) en forme de palier pour le palier de rotation et de frein (B$_6$), et en ce que l'élément de support (71) se règle par rapport à l'axe (A$_6$) par une première vis de réglage (73, 73') dans la direction horizontale (1) et par une seconde vis de réglage (74, 74') dans la direction verticale (2).

FIG. 2

FIG. 1